# EUROPEAN PATENT APPLICATION

(11) **EP 2 660 745 A2**
(43) Date of publication of application: **06.11.2013**
(21) Application number: 13165698.5
(22) Date of filing: 29.04.2013
(51) Int. Cl.: G06F 19/00

(54) **Mental health digital behavior monitoring system and method**

(30) Priority: 01.05.2012 US 201213460857
(71) Applicant: Almosni, Bernie, 47235 Ramat Hasharon (IL); Tal, Ilan, 69364 Israel (IL)
(72) Inventor: Almosni, Bernie, 47235 Ramat Hasharon (IL); Tal, Ilan, 69364 Israel (IL)
(74) Representative: Virdee-Crofts, Kulwinder Kaur

(57) **Abstract**

A system and method for monitoring a user's mental health. The user's use of electronic devices is tracked, such as usage of his mobile phone, tablet and his web activity. The invention "learns" each patient's unique behavioral patterns to be used as a "base line" representing the steady state (chronic phase) of the patient. The algorithmic processing unit detects any irregularities in a patient's behavioral patterns and produces a deterioration prediction. If it is determined that a threshold is exceeded, an alert is sent to a health professional.

## Description

### FIELD OF THE INVENTION

The present invention generally relates to mental health systems, and more particularly, the present invention provides a mental health digital behavior monitoring system and method.

### BACKGROUND OF THE INVENTION

One of the major problems in today's psychiatry common practice is the follow-up on patients; it is difficult to achieve fluent monitoring on a patient's condition. The result is low adherence to the treatment, either via medication or psychotherapy or both. During acute conditions patient tend to change dosage without consulting the therapist. However, after they feel better they do not relate to the treatment as a preventive measure, therefore stopping it. The low adherence rate for drugs or other treatments, leads to more episodes. Many times, patients do not seek treatment again until the next episode is disabling to the full extent, and requires major intervention.

Currently, maintaining follow-up on a patient is based mainly on face to face contact, adding short telephone conversations or emails. This kind of connection is limited and doesn't allow bidirectional flow of information in a fluent manner. The therapist (the psychiatrist, psychologist or any other mental health professional) is unable to really monitor the patient's condition between appointments. The problem worsens when the patient is in remission. Then, the therapist has no contact whatsoever with the patient, and most patients will stop treatment without notifying the therapist. The result is that mental health illnesses are moving towards being the primary cause of disability worldwide.

Currently psychiatrists, therapists and physicians cannot properly monitor drug adherence or assignments, such as those given in cognitive behavioral therapies, which are the key to the therapy's success. The therapist is also unaware of the patient's clinical condition, e.g., general mood, sleep quality, etc. Lack of regular monitoring on a mental health patient leads directly to more hospitalizations, disability and frequent visits, since the only way today to retrieve a mental health patient's clinical measures is by direct interview. Furthermore this problem tends to worsen due to the growing gap between the number of trained mental health professionals available and the population's needs.

Prior art for remote mental health monitoring typically require a patient to perform an activity such as filling out a questionnaire describing his recent movements. In effect, a patient undergoing even a subtle psychiatric episode may be unwilling or unable to comply, rendering these methods ineffective for the most part.

Prior art patent publications include:
US2011/118555: "System And Methods For Screening, Treating, And Monitoring, Psychological Conditions". A system for and method is disclosed for remote monitoring, screening, assessment and treatment of patients having a mental health illness such as post-traumatic stress disorders or other traumatic stress injury and co-occurring symptomatology. Patients in constant communication with one or more healthcare professionals through a wireless network, complete executable programs on their patient handheld electronic devices and transmit the results of the executable programs to their supervising mental health professional. The mental health professionals review and analyze the collected patient data to make clinical assessments of the patients' mental health status.
US7958228: "Behavioral Predictions Based On Network Activity Locations". A computer-implemented method is taught for constructing network activity profiles, which comprises the following: obtaining a plurality of records of network activities from an activity source, each record corresponding to an interaction with a network resource via the network from the activity source, wherein each record comprises at least a network endpoint address from where the interaction originates and an indication of a time of the interaction for each record, determining a geographical location corresponding to the network endpoint address of that record and associating the determined geographical location with that record and constructing at least one profile for the activity source based on the plurality of records and at least one geographical location associated with the records, wherein each profile comprises a time-based behavior pattern associated with the at least one geographical location.

The Diagnostic and Statistical Manual of Mental Disorders (DSM) and Common Clinical Practice rely on behavioral patterns to make a diagnosis, and furthermore, varieties of behavior are used to estimate patient's current condition, such as occupational and social functioning, concentration as expressed by work efficacy, etc. monitoring patients.

It would be advantageous to develop a scientific technology-based tool which can monitor a patient's well-being without requiring any action by a patient. This "background" monitoring is unique and especially effective, as patients in manic or depressed states may be unable or unwilling to comply with any monitoring that would require patient activity.

### SUMMARY OF THE INVENTION

It is not known in the art of psychiatry to monitor a patient in the background of his normal day, unknown to him and without requiring his active participation, using modern technology such as cellular phone usage and social networking usage. The present invention utilizes novel software that identifies a user's digital footprint and estimates a patient's current mental condition based on significant changes to a user's usual digital activity (social networking and cellular phone usage). The software produces a report and generates a response accordingly.

Accordingly, it is a principal object of the present invention to apply a digital footprint to monitor a patient's current condition.

It is another principal object of the present invention to use available technologies in day-to-day life, such as PC usage, Smartphone usage and different kinds of web activities such as Blogging, Social Networking, etc., in order to monitor the patient's behavioral patterns.

It is one other principal object of the present invention to provide a fluent online mental status update on a patient's behavior to the physician/therapist.

It is one further principal object of the present invention to provide a system that will "learn" each patient's unique and routine behavioral patterns.

It is one further principal object of the present invention to provide a system that will identify changes in the patient's behavioral patterns.

It is yet another principal object of the present invention to enable future predictions regarding the patient's condition and risk probability of developing various mental episodic conditions.

A mental health digital behavior monitoring support system is disclosed including Software Agent(s) to monitor and collect data concerning digital behaviors, such as, but not limited to, phone activity, web activity, personally generated network traffic and location patterns (location services). Behaviors are monitored by a software agent installed on a Smartphone, mobile phone, PC, tablet, or a software agent installed on a remote server configured to monitor Software as a Service (SaaS) solutions - such as web based e-mail accounts (e.g. Hotmail, Gmail), social network activity(e.g. Facebook, Linkedln), other kinds of web activity - such as blogging and recreational activity - such recreational activity may include any form of web browsing audio/video consumption such as YouTube, Netflix, Pandora, iTunes and similar new applications as they appear, including any kind of measurable content. Measurable content includes all forms of audio, visual, text and data.

Each agent resides on the device generating the data being monitored (such as an application for Smartphone or PC) in exception of agents dedicated of tracking SaaS solutions or web activities which will reside on a remote server. Each agent collects data from a specific device or data source and sends it in its raw format to an application server or service façade. The system also includes System Management Services responsible for correlating tasks between the different parts of the system. The application server may include a service broker that will transform the data into a single format that can be understood by the entire system. The transformed data will be saved to a storage that is maintained in a database management system such as an RDBMS or NoSQL system. The system also includes an Algorithmic Processing Unit to analyze each patient's data thus "learning" each patient's unique behavioral patterns. At some point the system will formulate a "base line" representing a steady state of the patient. The system continuously evaluates backwards in order to detect irregularities in the patient's behavioral patterns, most likely to occur when a patient is in the chronic phase. In the acute phase the system evaluates behavior for future comparison.

The system might be aware that a patient is in an acute phase, and will thereupon "learn" representative patterns accordingly. Such learning is applicable to improve results regarding probability for deterioration.

The inventors have noted changes in cellular phone or PC use that can occur in various mental disorders or situations (termed "psychiatric episodes"). Table I lists some exemplary correlations for personal computer (PC) related activities. Table II lists some exemplary correlations for phone-related activities. Table III lists some exemplary correlations for electronic mail and mobility related activities.

As is clear from Tables I-II, changes from a baseline pattern can be noted in any of the following characteristics of use of an electronic device: the time of day when usage occurs; the duration of usage; the frequency of said usage; the variety of usage; the destination of said usage; the content of said usage; identification of specific keywords; and the location of a user.

The changes may be associated with a wide variety of psychiatric disorders, including the following non-limiting examples shown in Tables I-III:
MDE, Manic, Dysthymic, Anorectic, Bulimic, Psychotic, Obsessive- compulsive, Panic, Phobic, GAD, Dissociative, Hypochondriac, PTSD, BLPD, Substance Abuse, Sleep disorders.

**Table I Changes in PC activity During a Psychiatric Episode**

| **Diagnosis-Episodes** | **PC general use** | **PC I/O activity** | **Avg. time on activity** | **Length of visit in site** | **Variety of software used** |
|---|---|---|---|---|---|
| **MDE** | D | D | I | I | D |
| **Manic** | I | I | D | D | I |
| **Dysthymic** | Same as MDE but milder | | | | |
| **Anorectic** | N | N | N | N | N |
| **Bulimic** | N | N | N | N | N |
| **Psychotic** | E | E | E | E | E |
| **Obsessive-compulsive** | I or D or N | I or D or N | I | I | D |
| **Panic** | N | N | I | I | N |
| **Phobic** | N | N | N | N | N |
| **GAD** | N | N | I | N | N |
| **Dissociative** | N | N | N | N | N |
| **Hypochondriac** | N | N | N | N | N |
| **PTSD** | N | N | N | N | N |
| **BLPD** | E | E | E | E | E |
| **Substance Abuse** | N | N | N | N | N |
| **Sleep** | Use late night or early morning | Use late night or early morning | N | N | Use late night or early morning |

**Table II Changes in Phone Use During a Psvchiatric Episode**

| **Diagnosis-Episodes** | **Phone calls duration** | **Time when call is placed** | **destination** | **SMS destination** | **SMS frequency and content** |
|---|---|---|---|---|---|
| **MDE** | D | Early morning, late night | D | D | D |
| **Manic** | D | Late hours | I | I | I |
| **Dysthymic** | Same as MDE but milder | | | | |
| **Anorectic** | N | N | N | N | Relevant Keywords |
| **Bulimic** | N | N | N | N | Relevant Keywords |
| **Psychotic** | E | E | E | E | E, basic language mistakes |
| **Obsessive-compulsive** | I | N | N | N | SMS content should be longer and repetitive |
| **Panic** | I | N | Frequent same destinations, esp. to close family members | Frequent same destinations, esp. to close family members | Keywords |
| **Phobic** | N | N | N | N | Keywords |
| **GAD** | N | N | N | N | Keywords |
| **Dissociative** | N | N | N | N | |
| **Hypochondriac** | N | N | N | N | Keywords |
| **PTSD** | N | Late Night | | N | Keywords |
| **BLPD** | E | E | IA to certain destinations, than DA | IA to certain destinations, than DA | Keywords |
| **Substance Abuse** | Varies | Before relapse | SC | Varies | Keywords |
| **Sleep** | N | Late Night | N | N | Keywords |

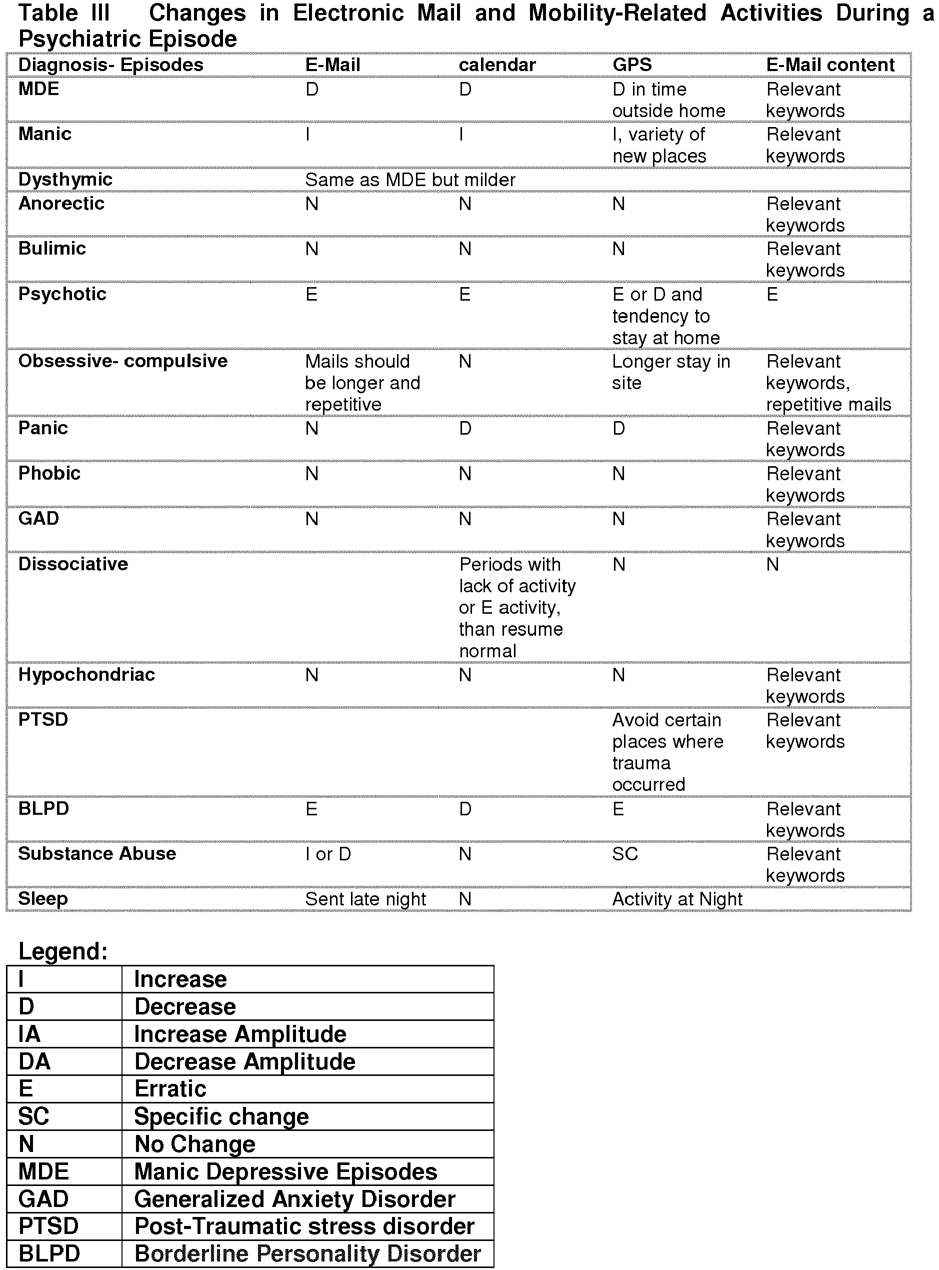

All the above and other characteristics and advantages of the invention will be further understood through the following illustrative and non-limitative description of preferred embodiments thereof.

### BRIEF DESCRIPTION OF THE DRAWINGS

In order to understand the invention and to see how it may be carried out in practice, a preferred embodiment will now be described, by way of a non-limiting example only, with reference to the accompanying drawings, in the drawings:
Fig. 1 is a schematic illustration showing how different input devices are used to monitor the patient, constructed according to the principles of the present invention;
Fig. 2 is a detailed schematic illustration showing the system's architecture and how the parts of the system are combined and used to monitor the patient and process the information obtained, constructed according to the principles of the present invention;
Fig. 3 is a general flow chart representation of the method for implementing the mental health digital behavior monitoring support system, constructed according to the principles of the present invention;
Fig. 4 is a detailed flow chart representation of the method for implementing the mental health digital behavior monitoring support system, constructed according to the principles of the present invention; and
Fig. 5 is graphical illustration of the chronic and acute phases of the mental health digital behavior monitoring support system, constructed according to the principles of the present invention.

### DETAILED DESCRIPTION OF PREFERRED EMBODIMENTS

The principles and operation of a method and an apparatus according to the present invention may be better understood with reference to the drawings and the accompanying description, it being understood that these drawings are given for illustrative purposes only and are not meant to be limiting.

The inventors have noted changes in cellular phone or PC use that can occur in various mental disorders or situations (termed "psychiatric episodes"). These include changes in activity related to: electronic mail, cellular phone use (including short messages MMS or SMS), social networking, and mobility related activities (tracking of a user location using GPS tracking).

As shown in relation to Tables I-II, changes from a baseline pattern can be noted in any of the following characteristics of use of an electronic device: the time of day when usage occurs; the duration of usage; the frequency of said usage; the variety of usage; the destination of said usage; the content of said usage; identification of specific keywords; and the location of a user.

The changes may be associated with a wide variety of psychiatric disorders.

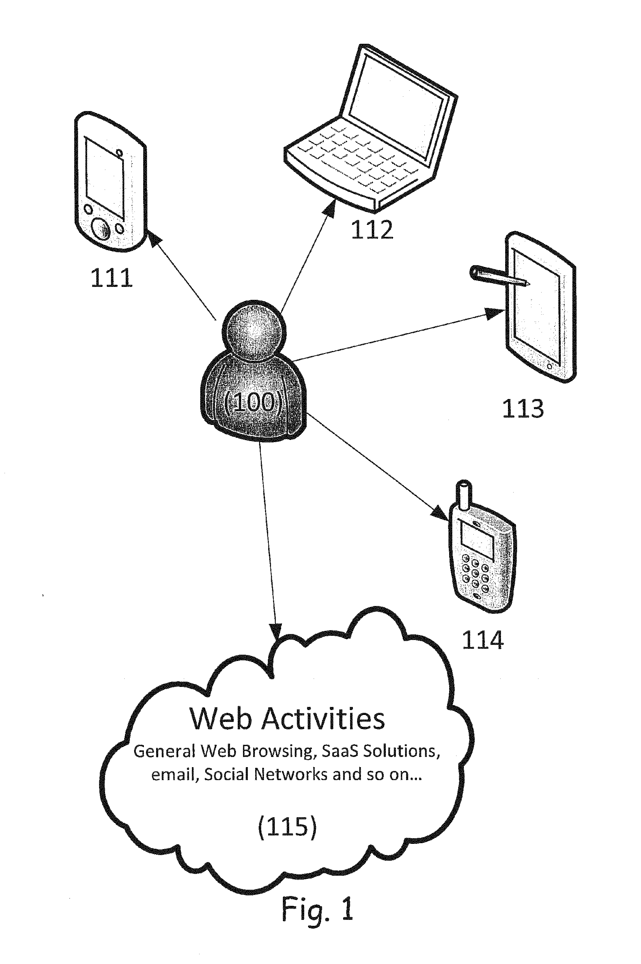

Fig. 1 is a general schematic illustration showing how different input devices from the patient's day to day digital life are used to monitor the patient's behavior 100, constructed according to the principles of the present invention. The system application provides Software Agent(s) in order to collect data from patient's Web Activities, such as general Web browsing, use of SaaS solutions (web based email for instance), social networks, personal blog, etc. 115 and from different kinds of input devices concerning the patient's digital life, such as Smartphone (personal digital assistant PDA) usage 111 and mobile phone usage 114, PC 112 and tablet 113 usage 112, or a laptop (not shown) as is clear to one skilled in the art.

Any input device in patient's digital life can produce "Web Activities:". A person can be active on the web for example using his Facebook account, from different input devices. He can use his PC browser, a dedicated PC Client application or a Smartphone application in order to use "Facebook." As for tracking his activities on "Facebook," one doesn't need a dedicated software agent for each of the platforms from which he accesses his Facebook Account. The system can use the patient's credentials (username and password) and gain access to the patient Facebook account.

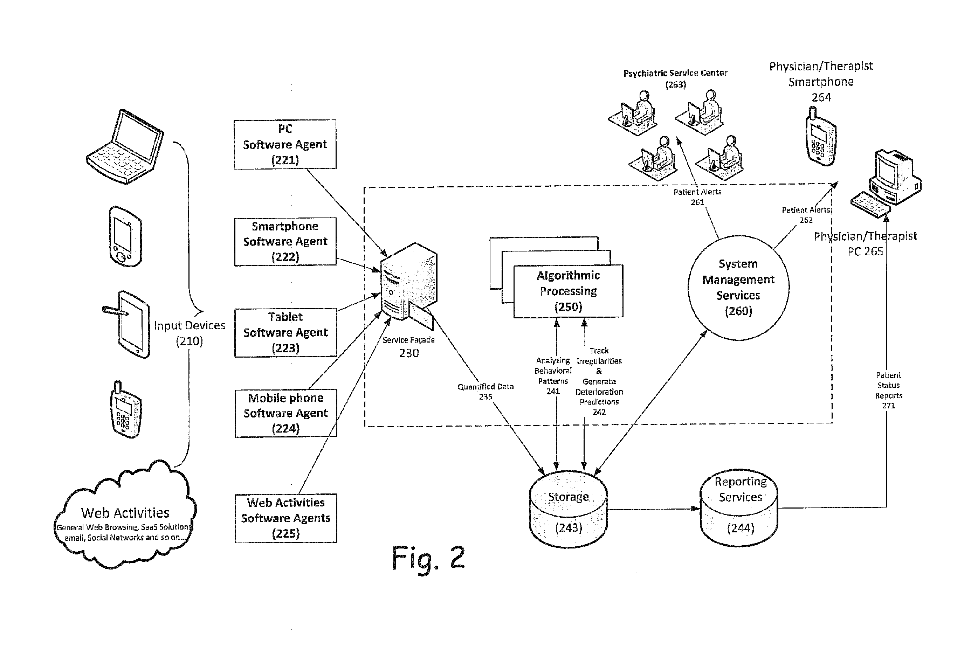

Fig. 2 is a detailed schematic illustration showing the system's architecture and how the parts of the system are combined and used to monitor the patient and process the information obtained, constructed according to the principles of the present invention. As shown in Fig. 1 patient's digital life are manifested by conducting various web activities and using different input devices 210. The activity data is collected and a user's activity is tracked by dedicated software agents 221 to 225 and sent to a data processing server - the service façade 230, which will quantify and store the data 235. The data will be analyzed using artificial intelligence algorithms 250, (wherein in an exemplary embodiment the service façade, algorithms and system management services reside in the same place) such as machine learning, pattern recognition, artificial neural networks and predictive analysis, in order to track irregularities and generate deterioration predictions 242. The system will allow access to patient status reports for his physician/therapist 271 and send alerts 262 to a physician/therapist Smartphone 264 or PC 265, for example, regarding the patient's condition and risk probability of developing any mental condition, especially a risky one.

### Software Agents

Different kinds of software agents 221 to 225 will be used in order to collect data from various inputs in patient's day to day digital life. Each agent, 221 to 225 collects data from a certain device or data source and sends it in a raw format to the Service façade 230.

### PC Software Agents

PC software agents 221 that monitor a patient's PC activity enable various opportunities to monitor the patient's behavior on the PC. Such as:
- General User Activity
   o Login / Logout.
   o Idle Time.
- I/O Activity
   o Network traffic (how many bytes are sent and received from the PC).
   o Files Created/Modified/Deleted.
- Use of Applications on PC
   Some applications have a certain meaning from the patient functional level perspective.
   o Office Applications (Such as Word or Excel).
   o Multimedia Applications.
   o Games.
- Browser Activity Monitoring.
   o Which web pages are being visited
   o How long the patient is staying on the same site.

### Smartphone Software Agents

Smartphone software agents 222 will reside on the Smartphone itself they will monitor different activities on the Smartphone, such as:
● Location Services
● Phone calls
● Text Messaging
● Data transfer and consumption
● General Use (Idle vs. Active times).

### Tablet Software Agents

Tablet Software Agents 223 will reside on a tablet. Tablets are can be considered as highly mobile PC's therefore a tablet software agent will be a combination between the PC and Smartphone agents.

### Mobile Phone Software Agents

Mobile phone software agents 224 will reside on the mobile phone itself they will monitor simple activities on the mobile phone, such as:
● Phone calls
● Text Messaging
● Data transfer and consumption.

### Web Activities Software Agents 225:

Web activities software agents are dedicated service applications operating from a remote server 225 these software agents run schedulers that from time to time access a certain account of the patient to track the patient's activities on that particular account.

### Social Networks Software Agents

Social Networks software Agents will monitor activity of a certain social network account, for Instance:
● Status Line updates
● Changes to Social Network (Adding friends, family members, colleagues and so on...)
● Profile Changes
● Other kinds of communications done through the account.
● General account activity (how frequently the account is visited for instance)
● 3rd Party application activity on the social network platform

### Email Software Agents

Email software agents will monitor activity on a certain email account, for instance:
● Correspondence
● Destination / Origin of messages.
● Frequency
● Content Analysis (searching keyword indicating deterioration).

### Web Parsing Agents

Web parsing agents are agents that monitor a certain activity on a certain web application or web site mostly meant for social networking activity (e.g. Facebook, Linkedln) and blogosphere activity. A custom agent will be designated for each platform, e.g., Facebook, Linkedln and blogosphere activity etc., the agent will monitor the patient's activity on that platform and also attempt to analyze meaningful content (searching keyword indicating deterioration). A web parsing agent will be necessary in case the system doesn't have patient's credentials to the type of activity it's attempting to monitor.

### Service Façade 230

Data inputs will arrive from various kinds of devices and formats. Therefore the system requires a mechanism that will receive all the data in one place and "normalize" the data into a single format that the system can understand and work with. Transformation of data (such as ETL, Extract Transform and Load) is a technique well-known to someone skilled in the art. The data arriving at service façade 230 will be re-formatted and quantified then the re-formatted data 235 will be stored in the systems storage 243.

### Algorithmic Processing Units 250

An Algorithmic Processing Unit's purpose is to analyze each patient's data thus formulating individual behavioral patterns. Basically the system will "learn" each patient unique behavioral pattern. Once the system has established the behavioral patterns for a certain patient, it will use it as a "base line" representing the steady state chronic phase of the patient, as well as a reference for the acute phase. Since human behavior is subject to change, the system will perform constant adjustments of this "base line." "Learning" will generally involve at least some (but not limited to) of the following technologies well-known to those skilled in the art:
● Machine Learning
● Pattern recognition
● Predictive Analysis

Some of these technologies will be used by another kind of algorithmic processing units which purpose is to detect irregularities and generate deterioration predictions based on a certain probability.

### System Management Services 260

System Management Services 260 is one core of the system. These services are responsible for correlating tasks between the different parts of the system. For instance, scheduling tasks for algorithmic processing 250 and managing notifications based on configuration, i.e., who should be receiving patient alerts 261, 262 in case of an event. Both System Management Services 260 and Reporting Services 244 receive data from storage 243.

### Reporting Services 244

Reporting Services 244 is the source from which reports are generated to the physician/therapist or the psychiatric service center.

### Storage 243

Storage 243 is maintained in a database management system, for example in a Relational database management system (RDBMS) or NoSQL system, Storage will be subject to regulations according Electronic Medical Records (EMR's). In computing, NoSQL is a class of database management system identified by its non-adherence to the widely-used relational database management system (RDBMS) model.

### Psychiatric Service Center 263

When the software providing System Management Services 260 identifies an event in which the patient's mental status has probably changed; it will initiate various warnings, one of which will be to a psychiatric service center 263. There, a mental health professional will be able to decide, according to the clinical data available to him, how to proceed and what is the level of urgency.

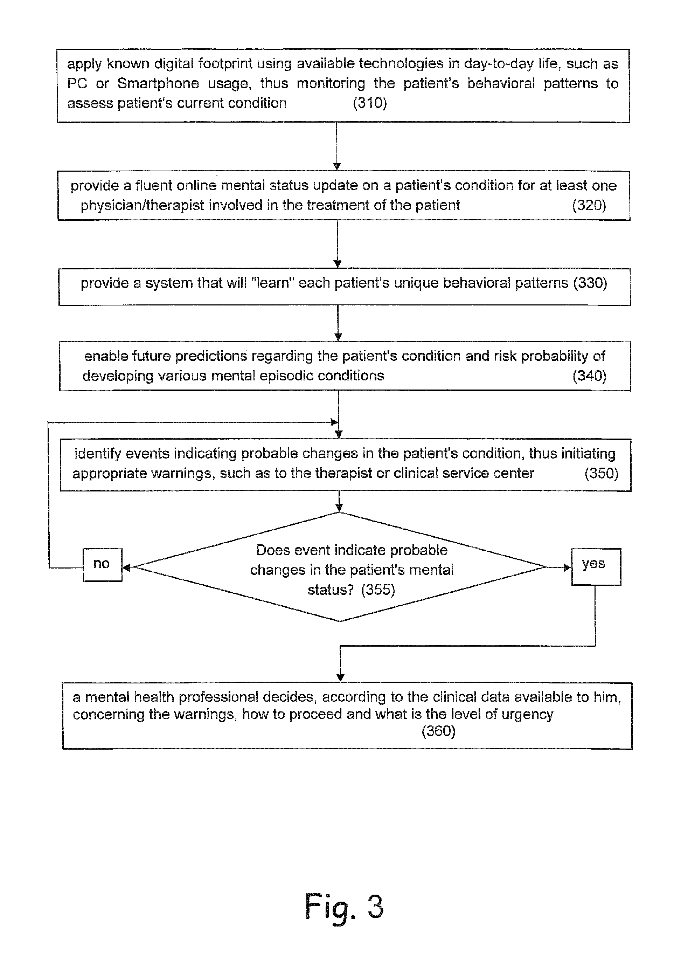

Fig. 3 is flow chart representation of the method for implementing the mental health digital behavior monitoring support system, constructed according to the principles of the present invention. First, identify a digital footprint, using input devices typical of modern day-to-day life, such as PC or Smartphone, in order to monitor the patient's behavioral patterns to assess patient's current condition 310. Then provide a fluent online mental status update on a patient's condition for at least one physician/therapist involved in the treatment of the patient 320 and provide a system that will "learn" each patient's unique behavioral patterns 330.

Next, using learning technologies listed above, enable future predictions regarding the patient's condition and risk probability of developing various mental episodic conditions 340 and identify events indicating probable changes in the patient's condition, thus initiating appropriate warnings, such as to the physician/therapist or clinical service center 350. Determine whether the event indicates probable changes in the patient's mental status 355, if the determination indicates that changes are probable, then a mental health professional decides, according to the clinical data available to him, concerning the warnings, how to proceed and what is the level of urgency 360. If the determination at step 355 determines that the event does not indicate probable changes in the patient's mental status 355, then continue to monitor and identify events according to reference block 350.

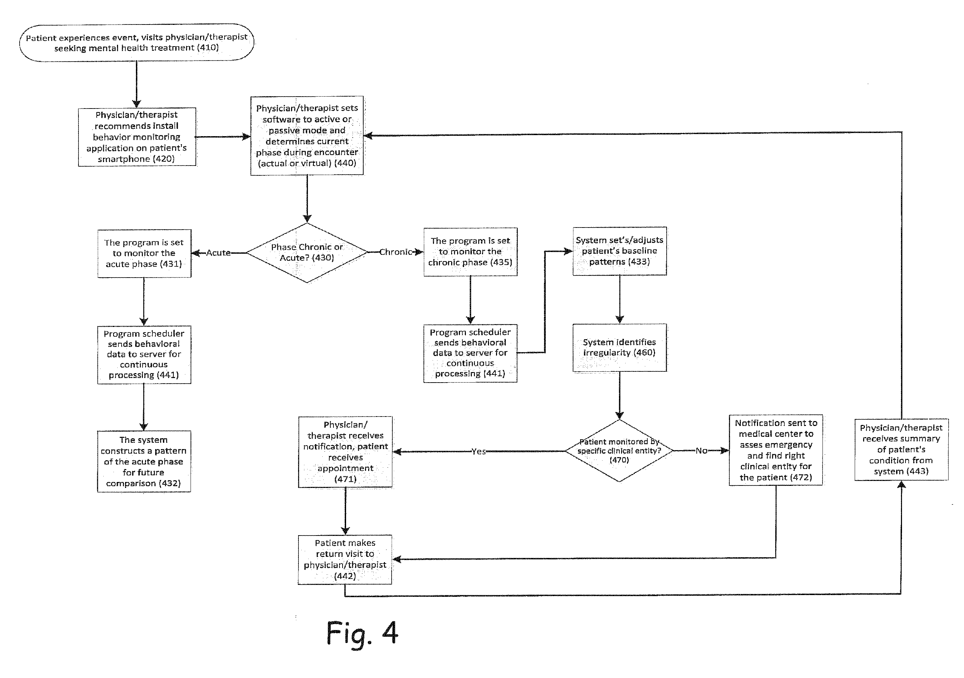

Fig. 4 is a detailed flow chart representation of the method for implementing the mental health digital behavior monitoring support system, constructed according to the principles of the present invention. When a patient experiences an event he may visit a physician or a therapist seeking mental health treatment 410. The physician/therapist preferably may recommend to the patient or to his caretaker, that they install the behavior monitoring application of the present invention on the patient's Smartphone 420 (or PC, shown in relation to Fig.1).

The physician/therapist sets software to active or passive mode. In an active mode the application will monitor patients behavior and will also provide bi-directional interaction between the patient and the physician/therapist (for instance physician can configure the application to pop-up a question to the patient each morning asking the patient about the quality of his sleep, from the patient side he can ask for a special notification to be passed to the physician/therapist regarding a certain question or feeling he has).

In a passive mode the application will only monitor patient's behavior. The physician/therapist determines current phase (chronic or acute) during encounter, whether the encounter is actual (patient is visiting physician/therapist clinic) or virtual (over the phone) 440. The physician/therapist gives a diagnosis as to whether the event chronic or acute 430.

If the physician/therapist gives a diagnosis of acute, the program is set to monitor the acute phase 431 and the system constructs a pattern representative of the acute phase for future comparison 432. If the physician/therapist gives a diagnosis of chronic, the program is set to monitor the chronic phase 435 and the program scheduler sends behavioral data to the server for continuous processing 441. The system sets and constantly adjusts the patient's baseline patterns during the chronic phase 433. During this time system is constantly searching for irregularities in patient's behavioral patterns, in case system identifies irregularity 460, if the patient is monitored by a specific treatment source (physician/therapist) 470, then the physician/therapist is notified and the patient receives an appointment 471. If the patient is not monitored by a specific treatment source, then the medical center is notified to find a suitable physician/therapist for the patient 472. In either case the patient again visits a physician/therapist 442 and the physician/therapist receives a brief summary of the patient's condition from the system 443.

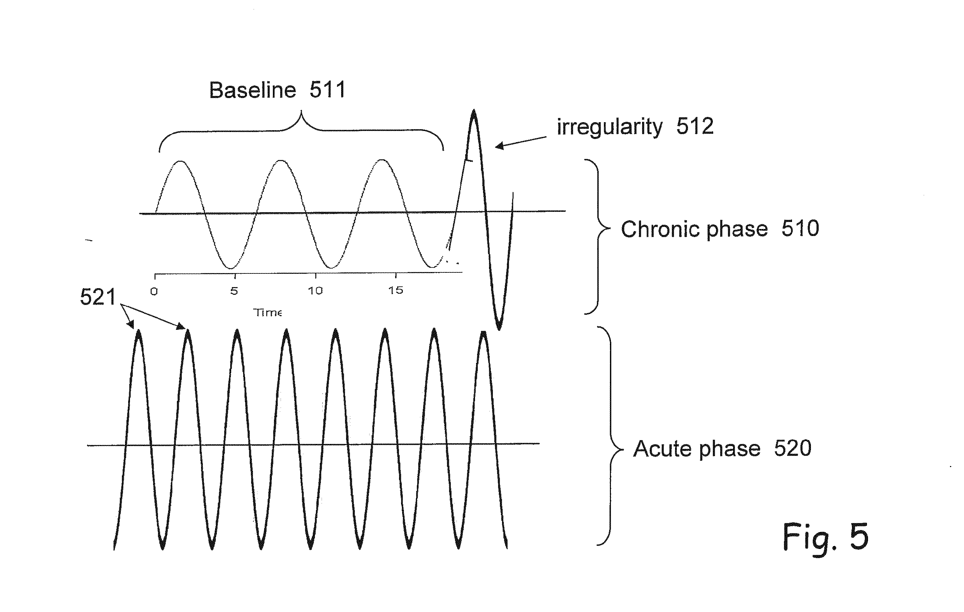

Fig. 5 is graphical representation of the chronic and acute phases of the mental health digital behavior monitoring support system, constructed according to the principles of the present invention. Chronic phase behavior 510 is characterized by steady-state baseline behavior 511, and ultimately by some form of irregularity 512. The spikes 521 of acute phase 520 resemble irregularity 512 of chronic phase 510, and are used as a basis for comparison. This figure illustrates how the system characterizes patterns and detects irregularities. This illustration doesn't correspond to any particular graphical representation of a certain mental illness or disorder.

Having described the present invention with regard to certain specific embodiments thereof, it is to be understood that the description is not meant as a limitation, since further modifications will now suggest themselves to those skilled in the art, and it is intended to cover such modifications as fall within the scope of the appended claims.

## Claims

1. A method for determining a user's metal health by tracking a user's digital activities, said method comprising:
a) tracking a user's use during a predetermined period, of at least one electronic device, and identifying a baseline pattern associated with a known user;
b) saving said baseline pattern in a database comprising user activities;
c) comparing a user's subsequent pattern of use of said at least one electronic device, with said saved baseline pattern;
d) algorithmically determining the significance of said comparison;
e) optionally, issuing a mental health alert to a medical professional if said comparison is determined to be significant.

2. The method of claim 1, wherein said at least one electronic device is selected from one or more of the following: a cellular phone, a personal computer, a tablet, a laptop, and a personal digital assistant.

3. The method of claim 1, wherein said baseline pattern comprises at least one of the following characteristics of use of an electronic device: the time of day when usage occurs; the duration of usage; the frequency of said usage; the variety of usage; the destination of said usage; the content of said usage; identification of specific keywords; and the location of a user.

4. The method of claim 1, wherein said use of said electronic device comprises one or more of the following uses: accessing of social network media; use of an electronic mail program; website browsing, calls made using a cellular phone; sending messages using a cellular phone; and tracking a user's location using GPS location of a user's cellular phone.

5. The method of claim 1, wherein said method is performed automatically without requiring continuous user activity.

6. The method of claim 1, wherein said method is used to identify a mental state selected from: MDE, Manic, Dysthymic, Anorectic, Bulimic, Psychotic, Obsessive-compulsive, Panic, Phobic, GAD, Dissociative, Hypochondriac, PTSD, BLPD, Substance Abuse, and a Sleep disorder.

7. A system for determining a user's metal health by tracking a user's digital activities, the system comprising:
a. a processor;
b. a memory holding instructions that, when executed by the processor, cause the processor to:
track a user's use during a predetermined period, of at least one electronic device, and identify a baseline pattern associated with a known user;
save said baseline pattern in a database comprising user activities;
compare a user's subsequent pattern of use of said at least one electronic device, with said saved baseline pattern;
algorithmically determine the significance of said comparison;
optionally, issue a mental health alert to a medical professional if said comparison is determined to be significant.

8. The system of claim 7, wherein said at least one electronic device is selected from one or more of the following: a cellular phone, a personal computer, a tablet, a laptop, and a personal digital assistant.

9. The system of claim 7, further comprising a remote server in electronic communication with said system, and said server comprises at least one of: a database of baseline patterns associated with a plurality of known users; a database of contact information associated with a plurality of known users; and a database of contact information associated with a plurality of medical personnel associated with said known users.

10. The system of claim 7, wherein said server performs any of the following actions:
identify a baseline pattern associated with a known user; algorithmically determine the significance of said comparison; and issue said mental health alert to a medical professional.

11. The system of claim 7, wherein said processor executes said instructions automatically without requiring continuous user activity.

12. The system of claim 7, comprising at least one the following components: an algorithmic processing unit for identifying said baseline pattern; a system management service for correlating tasks in said system; a service façade for converting data into a single format; an electronic activity software agent for tracking a user's use of an electronic device, and a reporting service component for issuing a mental health alert to a medical professional.
